# EUROPEAN PATENT SPECIFICATION

(11) **EP 4 013 352 B1**
(45) Date of publication and mention of the grant of the patent: **22.10.2025**
(21) Application number: 20757075.5
(22) Date of filing: 28.07.2020
(51) Int. Cl.: A61F 2/24

(54) **PROSTHETIC HEART VALVE HAVING AT LEAST TWO TYPES OF STRUTS**
HERZKLAPPENPROTHESE MIT MINDESTENS ZWEI ARTEN VON STREBEN
VALVULE CARDIAQUE PROTHÉTIQUE AYANT AU MOINS DEUX TYPES D'ENTRETOISES

(30) Priority: 13.08.2019 US 201962886281 P
(43) Date of publication of application: 22.06.2022
(73) Proprietor: Edwards Lifesciences Corporation, Irvine, CA 92614-5688 (US)
(72) Inventor: DVORSKY, Anatoly, 30889 Caesarea (IL)
(74) Representative: Eisenführ Speiser
(86) International application number: PCT/US2020/043854
(87) International publication number: WO 2021/030044

(56) References cited:
- WO-A1-2013/106585
- US-A1- 2018 344 456

## Description

### CROSS-REFERENCE TO RELATED APPLICATION

The present application claims the benefit of U.S. Provisional Application No. 62/886,281, filed August 13, 2019.

### FIELD

The present disclosure relates to implantable, mechanically expandable prosthetic devices, such as prosthetic heart valves, and to methods and delivery assemblies for, and including, such prosthetic devices.

### BACKGROUND

The human heart can suffer from various valvular diseases. These valvular diseases can result in significant malfunctioning of the heart and ultimately require repair of the native valve or replacement of the native valve with an artificial valve. There are a number of known repair devices (e.g., stents) and artificial valves, as well as a number of known methods of implanting these devices and valves in humans. Percutaneous and minimally-invasive surgical approaches are used in various procedures to deliver prosthetic medical devices to locations inside the body that are not readily accessible by surgery or where access without surgery is desirable. In one specific example, a prosthetic heart valve can be mounted in a crimped state on the distal end of a delivery apparatus and advanced through the patient's vasculature (e.g., through a femoral artery and the aorta) until the prosthetic heart valve reaches the implantation site in the heart. The prosthetic heart valve is then expanded to its functional size, for example, by inflating a balloon on which the prosthetic valve is mounted, actuating a mechanical actuator that applies an expansion force to the prosthetic heart valve, or by deploying the prosthetic heart valve from a sheath of the delivery apparatus so that the prosthetic heart valve can self-expand to its functional size.

Prosthetic heart valves that rely on a mechanical actuator for expansion can be referred to as "mechanically expandable" prosthetic heart valves. Mechanically expandable prosthetic heart valves can provide one or more advantages over self-expandable and balloon-expandable prosthetic heart valves. For example, mechanically expandable prosthetic heart valves can be expanded to various diameters. Mechanically expandable prosthetic heart valves can also be compressed after an initial expansion (e.g., for repositioning and/or retrieval).

US 2018/0344456 Al discloses a prosthetic valve, which can comprise a radially expandable and compressible frame, which can include a plurality of struts which are pivotally joined together without requiring individual rivets. In some embodiments, the struts are interwoven, and can be joined using integral hinges formed in the struts, such as by performing alternate cuts on the struts, bending the struts to form stopper tabs adjacent to joints and/or drilling holes in the struts to facilitate interconnecting struts at joints, or otherwise forming integral hinges and corresponding holes at junction points between the struts. In another embodiment, the frame comprises a plurality of inner struts and outer struts which are connected by a plurality of chains of interconnected rivets, avoiding the need to provide individual rivets at each junction between struts. In still another embodiment, separate hinges are provided to interconnect the struts. In still another embodiment, separate flanged rivets are provided to connect the struts.

Despite the recent advancements in percutaneous valve technology, there remains a need for improved transcatheter heart valves and delivery devices for such valves.

### SUMMARY

The invention is an implantable prosthetic device as defined by claim 1. In one representative embodiment, an implantable prosthetic device comprises a frame that is radially expandable and compressible between a radially compressed state and a radially expanded state. The frame comprises a first set of first struts and a second set of second struts. The frame has a distal end and a proximal end. The first struts are pivotably connected to each other at a plurality of distal and proximal apices at the distal and proximal ends of the frame, respectively. The second struts are pivotably connected to each other at a plurality of distal and proximal apices at the distal and proximal ends of the frame, respectively. The first struts are pivotably connected to the second struts at junctions between the distal and proximal ends of the frame. The first struts have a first thickness, which is measured between a radially facing inner surface and a radially facing outer surface of each first strut. The second struts have a second thickness, which is measured between a radially facing inner surface and a radially facing outer surface of each second strut. The first thickness is greater than the second thickness. One or more actuators are coupled only to one or more corresponding pairs of a distal apex and a proximal apex formed by the first struts, wherein the one or more actuators are configured to apply axially directed forces to the frame to radially expand the frame from the radially compressed state to the radially expanded state.

In some embodiments, the one or more actuators are configured to retain the frame in the radially expanded state.

In some embodiments, the one or more actuators comprise three actuators, each of which is coupled to a corresponding pair of a distal apex and a proximal apex formed by the first struts.

In some embodiments, the first struts are made of a first material, the second struts are made of a second material, and the first material is more rigid than the second material.

In some embodiments, the first struts are pivotably connected to each other at middle junctions located at midsections of the first struts, and the second struts are pivotably connected to each other at middle junctions located at midsections of the second struts.

In some embodiments, the frame comprises a third set of third struts, wherein the third struts are pivotably connected to each other at a plurality of distal and proximal apices at the distal and proximal ends of the frame, respectively. The third struts are pivotably connected to the first and second struts at junctions between the distal and proximal ends of the frame. The third struts have a third thickness, which is measured between a radially facing inner surface and a radially facing outer surface of each third strut. The first thickness is greater than the third thickness.

In some embodiments, the second thickness and the third thickness are the same.

In some embodiments, the first struts have a greater resistance against deformation from axially directed forces applied to the frame than the second struts.

In some embodiments, a plurality of leaflets are disposed in the frame and are configured to regulate a flow of blood through the frame in one direction.

In some embodiments, a fabric skirt is mounted to the frame with sutures extending through openings in the second struts.

In another representative embodiment, an implantable prosthetic device comprises a frame being radially expandable and compressible between a radially compressed state and a radially expanded state, the frame comprising a first set of first struts and a second set of second struts, the frame having a distal end and a proximal end. The first struts are pivotably connected to each other at a plurality of distal and proximal apices at the distal and proximal ends of the frame, respectively. The second struts are pivotably connected to each other at a plurality of distal and proximal apices at the distal and proximal ends of the frame, respectively. The first struts are pivotably connected to the second struts at junctions between the distal and proximal ends of the frame. The first struts have a greater resistance against deformation from axially directed forces applied to the frame than the second struts. One or more actuators are coupled only to one or more corresponding pairs of a distal apex and a proximal apex formed by the first struts, wherein the one or more actuators are configured to apply axially directed forces to the frame to radially expand the frame from the radially compressed state to the radially expanded state.

In some embodiments, the first struts have a first cross-sectional profile taken in a plane perpendicular to their length, the first cross-sectional profile having a first dimension measured in a direction. The second struts have a second cross-sectional profile taken in a plane perpendicular to their length, the second cross-sectional profile having a second dimension that is measured in the same direction as the first dimension of the first cross-sectional profile. The first dimension is greater than the second dimension.

In some embodiments, the first dimension is a first thickness, which is measured between a radially facing inner surface and a radially facing outer surface of each first strut. The second dimension a second thickness, which is measured between a radially facing inner surface and a radially facing outer surface of each second strut. The first thickness is greater than the second thickness.

In some embodiments, the first dimension is a first width, which is measured between first and second longitudinal edges of each first strut facing the distal and proximal ends of the frame, respectively. The second dimension is a second width, which is measured between first and second longitudinal edges of each second strut facing the distal and proximal ends of the frame, respectively. The first width is greater than the second width.

In some embodiments, the first struts are made of a first material, the second struts are made of a second material, and the first material is more rigid than the second material.

In some embodiments, the first struts comprise reinforcing ribs. In some embodiments, each first strut comprises one or more ribs that extend lengthwise of the first struts. In some embodiments, each first strut comprises a plurality of spaced-apart transverse ribs extending widthwise of the first struts.

In some embodiments, each of one or more actuators comprises first and second members axially moveable relative to each other, wherein the first member is pivotably coupled to a distal apex and the second member is pivotably coupled to a proximal apex formed by the first struts. In some embodiments, the first member is an outer member and the second member is an inner member that is partially received within the outer member.

In some embodiments, a plurality of leaflets disposed in the frame and configured to regulate a flow of blood through the frame in one direction.

In another representative embodiment, an implantable prosthetic device comprises a frame that is radially expandable and compressible between a radially compressed state and a radially expanded state, the frame comprising a first set of first struts, a second set of second struts, and a third set of third struts, the frame having a distal end and a proximal end. The first struts are pivotably connected to each other at a plurality of distal and proximal apices at the distal and proximal ends of the frame, respectively. The second struts are pivotably connected to each other at a plurality of distal and proximal apices at the distal and proximal ends of the frame, respectively. The third struts are pivotably connected to each other at a plurality of distal and proximal apices at the distal and proximal ends of the frame, respectively. The first struts are pivotably connected to the second and third struts at junctions between the distal and proximal ends of the frame. The second struts are pivotably connected to the first and third struts at junctions between the distal and proximal ends of the frame. The first struts have a greater resistance against deformation from axially directed forces applied to the frame than the second and third struts. One or more actuators are coupled only to one or more corresponding pairs of a distal apex and a proximal apex formed by the first struts, wherein the one or more actuators are configured to apply axially directed forces to the frame to radially expand the frame from the radially compressed state to the radially expanded state.

In some embodiments, the first struts have a first cross-sectional profile taken in a plane perpendicular to their length, the first cross-sectional profile having a first dimension measured in a direction. The second struts have a second cross-sectional profile taken in a plane perpendicular to their length, the second cross-sectional profile having a second dimension that is measured in the same direction as the first dimension of the first cross-sectional profile. The third struts have a third cross-sectional profile taken in a plane perpendicular to their length, the third cross-sectional profile having a third dimension that is measured in the same direction as the first dimension of the first cross-sectional profile. The first dimension is greater than the second dimension and the third dimension.

In some embodiments, the first dimension is a first thickness, which is measured between a radially facing inner surface and a radially facing outer surface of each first strut. The second dimension is a second thickness, which is measured between a radially facing inner surface and a radially facing outer surface of each second strut. The third dimension is a third thickness, which is measured between a radially facing inner surface and a radially facing outer surface of each third strut. The first thickness is greater than the second thickness and the third thickness.

In some embodiments, the first dimension is a first width, which is measured between first and second longitudinal edges of each first strut facing the distal and proximal ends of the frame, respectively. The second dimension is a second width, which is measured between first and second longitudinal edges of each second strut facing the distal and proximal ends of the frame, respectively. The third dimension is a third width, which is measured between first and second longitudinal edges of each third strut facing the distal and proximal ends of the frame, respectively. The first width is greater than the second width and the third width.

In some embodiments, the first struts are made of a first material, the second and third struts are made of a second material, and the first material is more rigid than the second material.

In some embodiments, the first struts comprise reinforcing ribs. In some embodiments, each first strut comprises one or more ribs that extend lengthwise of the first struts. In some embodiments, each first strut comprises a plurality of spaced-apart transverse ribs extending widthwise of the first struts.

In some embodiments, each distal and proximal apex and each junction comprise a pivot connector that allows the first, second, and third struts to pivot relative to each other when the frame is radially expanded and compressed.

In some embodiments, the device further comprises a plurality of leaflets disposed in the frame and configured to regulate a flow of blood through the frame in one direction.

In some embodiments, each of one or more actuators comprises first and second members axially moveable relative to each other, wherein the first member is pivotably coupled to a distal apex and the second member is pivotably coupled to a proximal apex formed by the first struts. In some embodiments, the first member is an outer member and the second member is an inner member that is partially received within the outer member.

In another representative embodiment, an implantable prosthetic device comprises a frame being radially expandable and compressible between a radially compressed state and a radially expanded state, the frame comprising a first set of first struts and a second set of second struts, wherein the first struts and the second struts are pivotably connected to each other by respective pivot connectors at a plurality of junctions, wherein the first struts have a greater resistance against deformation from axially directed forces applied to the frame than the second struts. One or more actuators are coupled only to one or more corresponding pairs of junctions formed by the first struts, wherein the one or more actuators are configured to apply axially directed forces to the frame to radially expand the frame from the radially compressed state to the radially expanded state.

In some embodiments, the frame has a distal end and a proximal end, wherein the first struts are pivotably connected to each other at junctions forming distal and proximal apices at the distal and proximal ends of the frame, respectively, and wherein the one or more actuators are coupled only to corresponding pairs of junctions forming distal and proximal apices of the frame.

In some embodiments, the frame has a distal end and a proximal end, and wherein the one or more actuators are coupled only to corresponding pairs of junctions that are axially spaced from the distal and proximal ends of the frame.

In some embodiments, the frame has a distal end and a proximal end, and wherein each of the one or more actuators are coupled to corresponding pairs of junctions that includes a junction at the distal or proximal end of the frame and a junction that is axially spaced from the distal and proximal ends of the frame.

In some embodiments, the first struts have a first cross-sectional profile taken in a plane perpendicular to their length, the first cross-sectional profile having a first dimension measured in a direction. The second struts have a second cross-sectional profile taken in a plane perpendicular to their length, the second cross-sectional profile having a second dimension that is measured in the same direction as the first dimension of the first cross-sectional profile. An average of the first dimension along the length of the first struts is greater than an average of the second dimension along the length of the second struts.

In some embodiments, the first dimension is a first thickness, which is measured between a radially facing inner surface and a radially facing outer surface of each first strut. The second dimension a second thickness, which is measured between a radially facing inner surface and a radially facing outer surface of each second strut. An average of the first thickness along the length of the first struts is greater than an average of the second thickness along the length of the second struts.

In some embodiments, the first thickness is constant along the length of the first struts and the second thickness is constant along the length of the second struts.

In some embodiments, the first thickness varies along the length of the first struts and the second thickness varies along the length of the second struts.

In some embodiments, the first dimension is a first width, which is measured between first and second longitudinal edges of each first strut facing the distal and proximal ends of the frame, respectively. The second dimension is a second width, which is measured between first and second longitudinal edges of each second strut facing the distal and proximal ends of the frame, respectively. An average of the first width along the length of the first struts is greater than an average of the second width along the length of the second struts.

In some embodiments, the first width is constant along the length of the first struts and the second width is constant along the length of the second struts.

In some embodiments, the first width varies along the length of the first struts and the second width varies along the length of the second struts.

In some embodiments, the first struts are made of a first material, the second struts are made of a second material, and the first material is more rigid than the second material.

In some embodiments, the first struts comprise reinforcing ribs. In some embodiments, each first strut comprises one or more ribs that extend lengthwise of the first struts. In some embodiments, each first strut comprises a plurality of spaced-apart transverse ribs extending widthwise of the first struts.

In some embodiments, each of one or more actuators comprises first and second members axially moveable relative to each other, wherein the first member is pivotably coupled to an adjacent junction and the second member is pivotably coupled to an adjacent junction. In some embodiments, the first member is an outer member and the second member is an inner member that is partially received within the outer member.

In some embodiments, the device further comprises a plurality of leaflets disposed in the frame and configured to regulate a flow of blood through the frame in one direction.

The foregoing and other objects, features, and advantages of the invention will become more apparent from the following detailed description, which proceeds with reference to the accompanying figures.

### BRIEF DESCRIPTION OF THE DRAWINGS

FIG. 1 is a perspective view of a prosthetic heart valve, according to one embodiment.
FIG. 2A is a side elevation view of the frame of the prosthetic heart valve of FIG. 1, shown in a radially compressed state.
FIG. 2B is a side elevation view of the frame of the prosthetic heart valve of FIG. 1, shown in a radially expanded state.
FIG. 3 is a perspective view of a prosthetic valve frame, shown in a radially collapsed state, having a plurality of expansion and locking mechanisms, according to another embodiment.
FIG. 4 is a perspective view of the frame and the expansion and locking mechanisms of FIG. 3, with the frame shown in a radially expanded state.
FIG. 5A is a perspective view of a screw of one of the expansion and locking mechanisms of FIG. 3.
FIG. 5B is a perspective view of one of the expansion and locking mechanisms of FIG. 3.
FIG. 5C is another perspective view of the frame and the expansion and locking mechanisms of FIG. 3, with the frame shown in a radially expanded state.
FIG. 6 is another perspective view of one of the expansion and locking mechanisms of FIG. 3.
FIG. 7 shows a cross sectional view of one of the expansion and locking mechanisms of FIG. 3 along with a portion of the frame.
FIG. 8 is side elevation view of a delivery apparatus for a prosthetic heart valve, according to one embodiment.
FIG. 9 is a perspective view of a frame for a prosthetic heart valve, according to another embodiment.
FIG. 10A is a top plan view of a first type of strut that is used to form the frame of FIG. 9.
FIG. 10B is a top plan view of a second type of strut that is used to form the frame of FIG. 9.
FIG. 11A is a side view of a first type of strut that is used to form the frame of FIG. 9.
FIG. 11B is a side view of a second type of strut that is used to form the frame of FIG. 9.
FIG. 12A is another embodiment of a type of reinforced strut that can be used to form the frame of FIG. 9.
FIG. 12B is a cross-sectional view of the strut of FIG. 12A taken along line 12B-12B.
FIG. 13 is another embodiment of a type of reinforced strut that can be used to form the frame of FIG. 9.
FIG. 14 is a side view of a portion of a frame, according to another embodiment.
FIG. 15 is a side view of a portion of a frame, according to another embodiment.

### DETAILED DESCRIPTION

Described herein are examples of prosthetic implants, such as prosthetic heart valves that can be implanted within any of the native valves of the heart (e.g., the aortic, mitral, tricuspid and pulmonary valves). The present disclosure also provides frames for use with such prosthetic implants. The frames can further comprise actuators and locking mechanisms to enable greater control over the radial compression or expansion of the valve body. The frames can comprise struts having different shapes and/or sizes to minimize the overall crimp profile of the implant and provide sufficient structural strength and rigidity to areas of the where needed.

Prosthetic valves disclosed herein can be radially compressible and expandable between a radially compressed state and a radially expanded state. Thus, the prosthetic valves can be crimped on or retained by an implant delivery apparatus in the radially compressed state during delivery, and then expanded to the radially expanded state once the prosthetic valve reaches the implantation site. It is understood that the valves disclosed herein may be used with a variety of implant delivery apparatuses, and examples thereof will be discussed in more detail later.

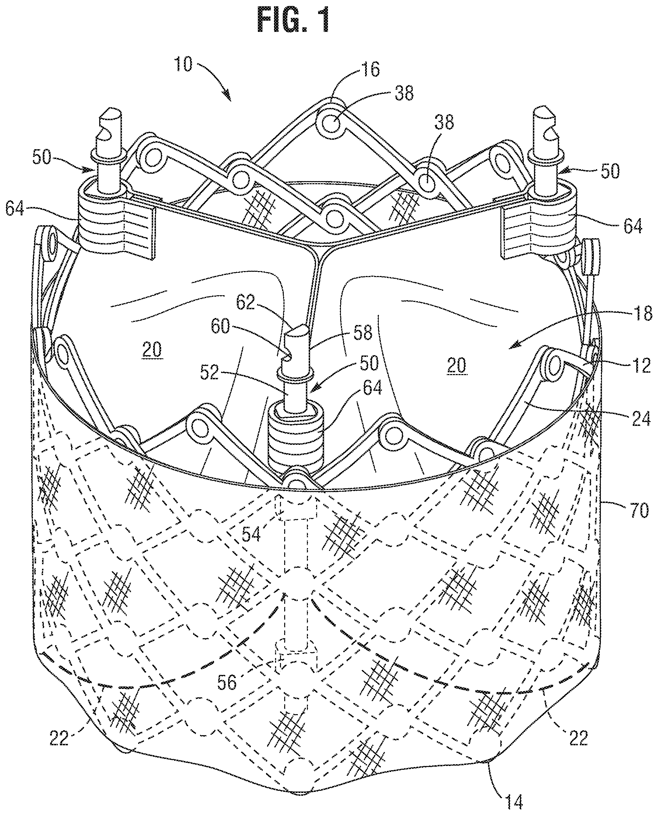

FIG. 1 shows an exemplary prosthetic valve 10, according to one embodiment. The prosthetic valve 10 can include an annular stent or frame 12 having an inflow end 14 and an outflow end 16. The prosthetic valve 10 can also include a valvular structure 18 which is coupled to and supported inside of the frame 12. The valvular structure 18 is configured to regulate the flow of blood through the prosthetic valve 10 from the inflow end 14 to the outflow end 16.

The valvular structure 18 can include, for example, a leaflet assembly comprising one or more leaflets 20 made of a flexible material. The leaflets 20 can be made from in whole or part, biological material, bio-compatible synthetic materials, or other such materials. Suitable biological material can include, for example, bovine pericardium (or pericardium from other sources). The leaflets 20 can be secured to one another at their adjacent sides to form commissures, each of which can be secured to a respective actuator 50 or the frame 102.

In the depicted embodiment, the valvular structure 18 comprises three leaflets 20, which can be arranged to collapse in a tricuspid arrangement. Each leaflet 20 can have an inflow edge portion 22. As shown in FIG. 1, the inflow edge portions 22 of the leaflets 20 can define an undulating, curved scallop shape that follows or tracks a plurality of interconnected strut segments of the frame 12 in a circumferential direction when the frame 12 is in the radially expanded configuration. The inflow edges of the leaflets can be referred to as a "scallop line."

In some embodiments, the inflow edge portions 22 of the leaflets 20 can be sutured to adjacent struts of the frame generally along the scallop line. In other embodiments, the inflow edge portions 22 of the leaflets 20 can be sutured to an inner skirt, which in turn in sutured to adjacent struts of the frame. By forming the leaflets 20 with this scallop geometry, stresses on the leaflets 20 are reduced, which in turn improves durability of the valve 10. Moreover, by virtue of the scallop shape, folds and ripples at the belly of each leaflet 20 (the central region of each leaflet), which can cause early calcification in those areas, can be eliminated or at least minimized. The scallop geometry also reduces the amount of tissue material used to form valvular structure 18, thereby allowing a smaller, more even crimped profile at the inflow end 14 of the valve 10.

Further details regarding transcatheter prosthetic heart valves, including the manner in which the valvular structure can be mounted to the frame of the prosthetic valve can be found, for example, in U.S. Patent Nos. 6,730,118, 7,393,360, 7,510,575, 7,993,394, and 8,252,202, U.S. Patent Application No. 15/978,459 (Published as U.S. Publication No. 2018/0325665) and U.S. Provisional Application No. 62/854,702, filed May 30, 2019.

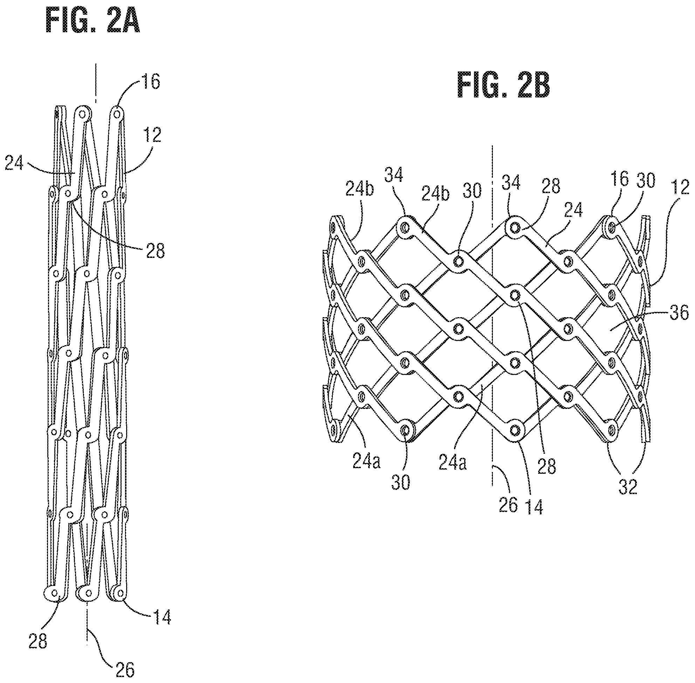

The prosthetic valve 10 can be radially compressible and expandable between a radially compressed configuration and a radially expanded configuration. FIGS. 2A-2B show the bare frame 12 of the prosthetic valve 10 (without the leaflets and other components) for purposes of illustrating expansion of the prosthetic valve 10 from the radially compressed configuration (FIG. 2A) to the radially expanded configuration (FIG. 2B).

The frame 12 can include a plurality of interconnected lattice struts 24 arranged in a lattice-type pattern and forming a plurality of apices 34 at the outflow end 16 of the prosthetic valve 10. The struts 24 can also form similar apices 32 at the inflow end 14 of the prosthetic valve 10. In FIG. 2B, the struts 24 are shown as positioned diagonally, or offset at an angle relative to, and radially offset from, a longitudinal axis 26 of the prosthetic valve 10 when the prosthetic valve 10 is in the expanded configuration. In other implementations, the struts 24 can be offset by a different amount than depicted in FIG. 2B, or some or all of the struts 24 can be positioned parallel to the longitudinal axis 26 of the prosthetic valve 10.

The struts 24 can comprise a set of inner struts 24a (extending from the lower left to the upper right of the frame in FIG. 2B) and a set of outer struts 24b (extending from the upper left to the lower right of the frame in FIG. 2B) connected to the inner struts 24a. The open lattice structure of the frame 12 can define a plurality of open frame cells 36 between the struts 24.

The struts 24 can be pivotably coupled to one another at one or more pivot joints or pivot junctions 28 along the length of each strut. For example, in one embodiment, each of the struts 24 can be formed with apertures 30 at opposing ends of the strut and apertures spaced along the length of the strut. Respective hinges can be formed at the locations where struts 24 overlap each other via fasteners 38 (FIG. 1), such as rivets or pins that extend through the apertures 30. The hinges can allow the struts 24 to pivot relative to one another as the frame 12 is radially expanded or compressed, such as during assembly, preparation, or implantation of the prosthetic valve 10.

The frame struts and the components used to form the pivot joints of the frame 12 (or any frames described below) can be made of any of various suitable materials, such as stainless steel, a cobalt chromium alloy, or a nickel titanium alloy ("NiTi"), for example Nitinol. In some embodiments, the frame 12 can be constructed by forming individual components (e.g., the struts and fasteners of the frame) and then mechanically assembling and connecting the individual components together. Further details regarding the construction of the frame and the prosthetic valve are described in U.S. Patent Publication Nos. 2018/0153689 and 2018/0344456, and U.S. Patent Application Nos. 16/105,353 and 62/748,284.

In the illustrated embodiment, the prosthetic valve 10 can be mechanically expanded from the radially contracted configuration to the radially expanded configuration. For example, the prosthetic valve 10 can be radially expanded by maintaining the inflow end 14 of the frame 12 at a fixed position while applying a force in the axial direction against the outflow end 16 toward the inflow end 14. Alternatively, the prosthetic valve 10 can be expanded by applying an axial force against the inflow end 14 while maintaining the outflow end 16 at a fixed position, or by applying opposing axial forces to the inflow and outflow ends 14, 16, respectively.

As shown in FIG. 1, the prosthetic valve 10 can include one or more actuators 50 mounted to and equally spaced around the inner surface of the frame 12. Each of the actuators 50 can be configured to form a releasable connection with one or more respective actuators of a delivery apparatus.

In the illustrated embodiment, expansion and compression forces can be applied to the frame by the actuators 50. Referring again to FIG. 1, each of the actuators 50 can comprise a screw or threaded rod 52, a first anchor in the form of a cylinder or sleeve 54, and a second anchor in the form of a threaded nut 56. The rod 52 extends through the sleeve 54 and the nut 56. The sleeve 54 can be secured to the frame 12, such as with a fastener 38 that forms a hinge at the junction between two struts. Each actuator 50 is configured to increase the distance between the attachment locations of a respective sleeve 54 and nut 56, which causes the frame 12 to elongate axially and compress radially, and to decrease the distance between the attachment locations of a respective sleeve 54 and nut 56, which causes the frame 12 to foreshorten axially and expand radially.

For example, each rod 52 can have external threads that engage internal threads of the nut 56 such that rotation of the rod causes corresponding axial movement of the nut 56 toward or away from the sleeve 54 (depending on the direction of rotation of the rod 52). This causes the hinges supporting the sleeve 54 and the nut 56 to move closer towards each other to radially expand the frame or to move farther away from each other to radially compress the frame, depending on the direction of rotation of the rod 52.

In other embodiments, the actuators 50 can be reciprocating type actuators configured to apply axial directed forces to the frame to produce radial expansion and compression of the frame. For example, the rod 52 of each actuator can be fixed axially relative to the sleeve 56 and slidable relative to the sleeve 54. Thus, in this manner, moving the rod 52 distally relative to the sleeve 54 and/or moving the sleeve 54 proximally relative to the rod 52 radially compresses the frame. Conversely, moving the rod 52 proximally relative to the sleeve 54 and/or moving the sleeve 54 distally relative to the rod 52 radially expands the frame.

When reciprocating type actuators are used, the prosthetic valve can also include one or more locking mechanisms that retain the frame in the expanded state. The locking mechanisms can be separate components that are mounted on the frame apart from the actuators, or they can be a sub-component of the actuators themselves.

Each rod 52 can include an attachment member 58 along a proximal end portion of the rod 52 configured to form a releasable connection with a corresponding actuator of a delivery apparatus. The actuator(s) of the delivery apparatus can apply forces to the rods for radially compressing or expanding the prosthetic valve 10. The attachment member 58 in the illustrated configuration comprises a notch 60 and a projection 62 that can engage a corresponding projection of an actuator of the delivery apparatus.

In the illustrated embodiments, the prosthetic valve 10 includes three such actuators 50, although a greater or fewer number of actuators could be used in other embodiments. The leaflets 20 can have commissure attachments members 64 that wrap around the sleeves 54 of the actuators 50. Further details of the actuators, locking mechanisms and delivery apparatuses for actuating the actuators can be found in U.S. Patent Publication No. 2018/0153689 and U.S. Patent Application Nos. 16/105,353, 15/831,197, 15/978,459, 63/013,912, 62/945,039, and 62/990,299. Any of the actuators and locking mechanisms disclosed in the previously filed applications can be incorporated in any of the prosthetic valves disclosed herein. Further, any of the delivery apparatuses disclosed in the previously filed applications can be used to deliver and implant any of the prosthetic valves discloses herein.

The prosthetic valve 10 can include one or more skirts or sealing members. In some embodiments, the prosthetic valve 10 can include an inner skirt (not shown) mounted on the inner surface of the frame. The inner skirt can function as a sealing member to prevent or decrease perivalvular leakage, to anchor the leaflets to the frame, and/or to protect the leaflets against damage caused by contact with the frame during crimping and during working cycles of the prosthetic valve. As shown in FIG. 1, the prosthetic valve 10 can also include an outer skirt 70 mounted on the outer surface of the frame 12. The outer skirt 70 can function as a sealing member for the prosthetic valve by sealing against the tissue of the native valve annulus and helping to reduce paravalvular leakage past the prosthetic valve. The inner and outer skirts can be formed from any of various suitable biocompatible materials, including any of various synthetic materials, including fabrics (e.g., polyethylene terephthalate fabric) or natural tissue (e.g., pericardial tissue). Further details regarding the use of skirts or sealing members in prosthetic valve can be found, for example, in U.S. Patent Application No. 62/854,702.

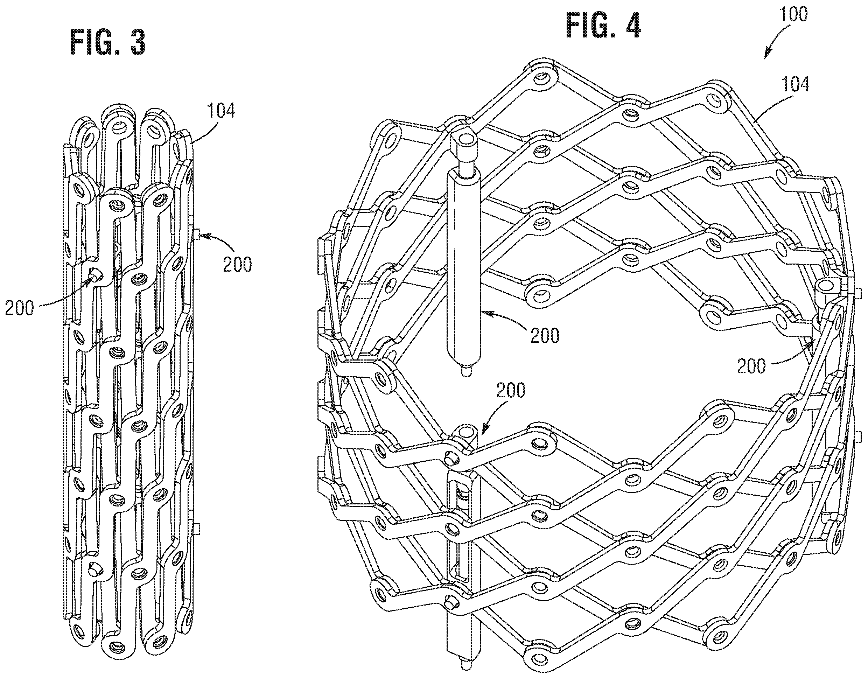

FIGS. 3-4 show another embodiment of a prosthetic valve 100 comprising a frame 104 and expansion and locking mechanisms 200 (also referred to as "actuators"). It should be understood that the prosthetic valve 100 can include leaflets 20 and other soft components, such as one or more skirts 70, which are removed for purposes of illustration. Expansion and locking mechanism 200 can be used to both radially expand and lock the prosthetic valve in a radially expanded state. In the example of FIGS. 3 and 4, three expansion and locking mechanisms 200 are attached to the frame 104 but in other example delivery assemblies, any number of expansion and locking mechanisms 200 can be used. FIG. 3 shows the expansion and locking mechanisms 200 attached to the frame 104 when the frame is in a radially collapsed configuration and FIG. 4 shows expansion and locking mechanisms attached to the frame when the frame is in a radially expanded configuration.

It will be appreciated that prosthetic valve 100 can, in certain embodiments, use other mechanisms for expansion and locking, such as linear actuators, alternate locking mechanisms, and alternate expansion and locking mechanisms. Further details regarding the use of linear actuators, locking mechanisms, and expansion and locking mechanisms in prosthetic valve can be found, for example, in U.S. Patent Publication No. 2018/0153689.

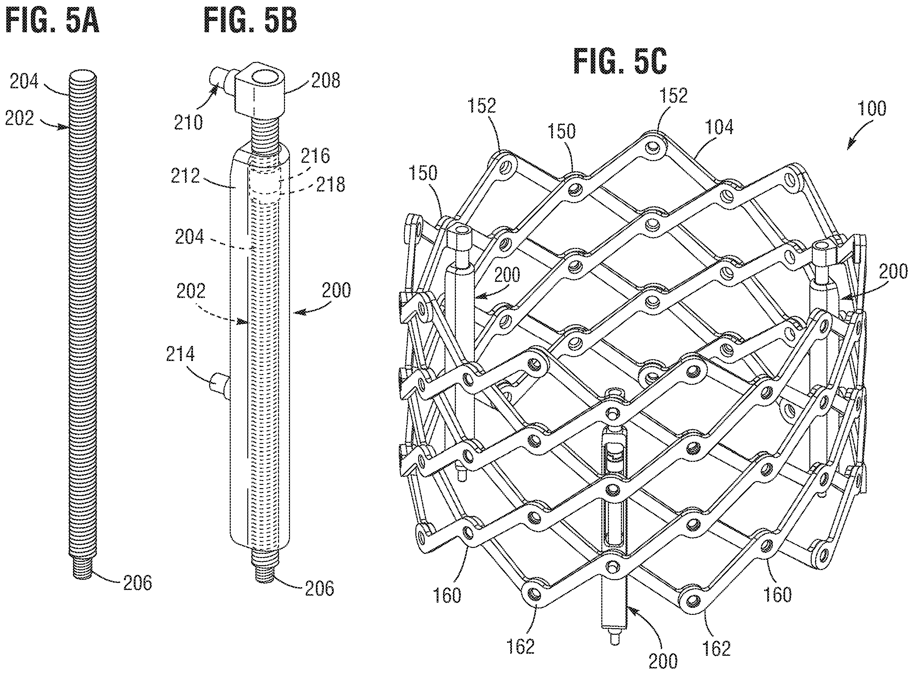

Referring to FIGS. 5A-5C, the expansion and locking mechanism 200 in the illustrated embodiment can include an actuator screw 202 (which functions as a linear actuator or a push-pull member in the illustrated embodiment) comprising a relatively long upper, or distal, portion 204 and a relatively shorter lower, or proximal, portion 206 at the proximal end of the screw 200, wherein the lower portion has a smaller diameter than the upper portion. Both the upper and lower portions 204, 206 of the screw 202 can have externally threaded surfaces.

The actuator screw 200 can have a distal attachment piece 208 attached to its distal end having a radially extending distal valve connector 210. The distal attachment piece 208 can be fixed to the screw 202 (e.g., welded together or manufactured as one piece). The distal valve connector 210 can extend through an opening at or near the distal end of the frame 104 formed at a location on the frame where two or more struts intersect as shown in FIG. 5C. The distal valve connector 210 can be fixed to the frame 104 (e.g., welded). Due to the shape of the struts, the distal end of the frame 104 comprises an alternating series of distal junctions 150 and distal apices 152. In the illustrated example, the distal valve connectors 210 of the three expansion and locking mechanisms 200 are connected to the frame 104 through distal junctions 150. In other examples, one or more distal valve connectors 210 can be connected to the frame 104 through distal apices 152. In other embodiments, the distal valve connectors 210 can be connected to junctions closer to the proximal end of the frame 104.

The expansion and locking mechanism 200 can further include a sleeve 212. The sleeve 212 can be positioned annularly around the distal portion 204 of the screw 202 and can contain axial openings at its proximal and distal ends through which the screw 202 can extend. The axial openings and the lumen in the sleeve 212 can have a diameter larger than the diameter of the distal portion 204 of the screw 202 such that the screw can move freely within the sleeve (the screw 202 can be moved proximally and distally relative to the sleeve 212). Because the actuator screw 202 can move freely within the sleeve, it can be used to radially expand and/or contract the frame 104 as disclosed in further detail below.

The sleeve 212 can have a proximal valve connector 214 extending radially from its outer surface. The proximal valve connector 214 can be fixed to the sleeve 212 (e.g., welded). The proximal valve connector 214 can be axially spaced from the distal valve connector 210 such that the proximal valve connector can extend through an opening at or near the proximal end of the frame 104. The proximal end of the frame 104 comprises an alternating series of proximal junctions 160 and proximal apices 162. In the illustrated example, the proximal valve connectors 214 of the three expansion and locking mechanisms 200 are connected to the frame 104 through proximal junctions 160. In other examples, one or more proximal valve connectors 214 can be connected to the frame 104 through proximal apices 162. In other embodiments, the proximal valve connectors 214 can be connected to junctions closer to the distal end of the frame 104.

It should be understood that the distal and proximal connectors 210, 214 need not be connected to opposite ends of the frame. The actuator 200 can be used to expand and compress the frame as long as the distal and proximal connectors are connected to respective junctions on the frame that are axially spaced from each other.

A locking nut 216 can be positioned inside of the sleeve 212 and can have an internally threaded surface that can engage the externally threaded surface of the actuator screw 202. The locking nut 216 can have a notched portion 218 at its proximal end, the purpose of which is described below. The locking nut can be used to lock the frame 104 into a particularly radially expanded state, as discussed below.

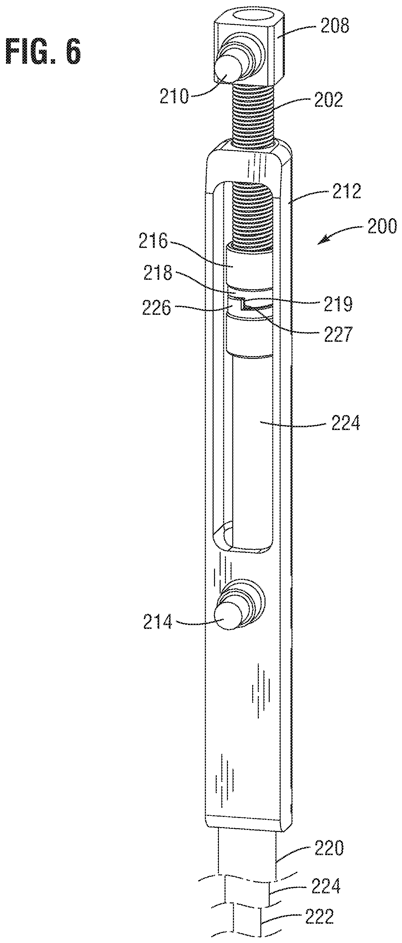

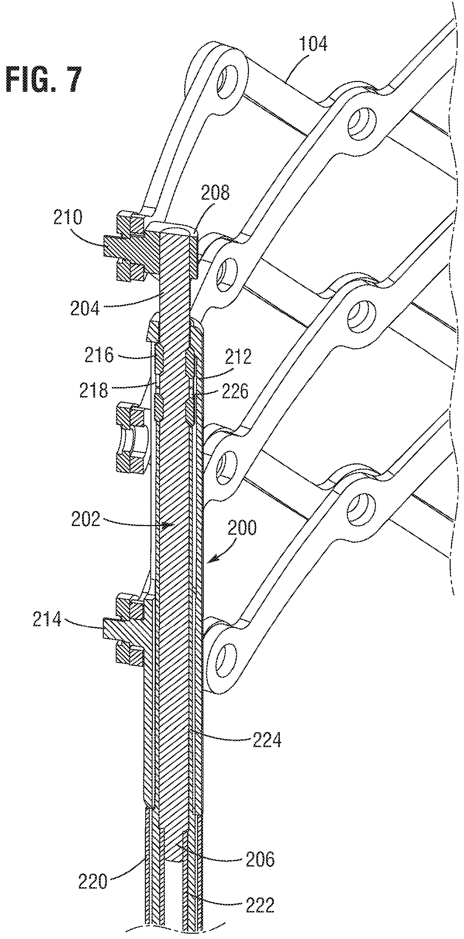

FIGS. 6 and 7 shows the expansion and locking mechanism 200 including components of a delivery apparatus not shown in FIGS. 5A-5C. As shown, the expansion and locking mechanism 200 can be releasably coupled to a support tube 220, an actuator member 222, and a locking tool 224. The proximal end of the support tube 220 can be connected to a handle or other control device (not shown) that a doctor or operator of the delivery assembly utilizes to operate the expansion and locking mechanism 200 as described herein. Similarly, the proximal ends of the actuator member 222 and the locking tool 224 can be connected to the handle.

The support tube 220 annularly surrounds a proximal portion of the locking tool 224 such that the locking tool extends through a lumen of the support tube. The support tube 220 and the sleeve are sized such that the distal end of the support tube abuts or engages the proximal end of the sleeve 212 such that the support tube is prevented from moving distally beyond the sleeve.

The actuator member 222 extends through a lumen of the locking tool 224. The actuator member 222 can be, for example, a shaft, a rod, a cable, or wire. The distal end portion of the actuator member 222 can be releasably connected to the proximal end portion 206 of the actuator screw 202. For example, the distal end portion of the actuator member 222 can have an internally threaded surface that can engage the external threads of the proximal end portion 206 of the actuator screw 202. Alternatively, the actuator member 222 can have external threads that engage an internally threaded portion of the screw 202. When the actuator member 222 is threaded onto the actuator screw 202, axial movement of the actuator member causes axial movement of the screw.

The distal portion of the locking tool 224 annularly surrounds the actuator screw 202 and extends through a lumen of the sleeve 212 and the proximal portion of the locking tool annularly surrounds the actuator member 222 and extends through a lumen of the support tube 220 to the handle of the delivery device. The locking tool 224 can have an internally threaded surface that can engage the externally threaded surface of the locking screw 202 such that clockwise or counter-clockwise rotation of the locking tool 224 causes the locking tool to advance distally or proximally along the screw, respectively.

The distal end of the locking tool 224 can comprise a notched portion 226, as can best be seen in FIG. 6. The notched portion 226 of the locking tool 224 can have an engagement surface 227 that is configured to engage a correspondingly shaped engagement surface 219 of the notched portion 218 of the locking nut 216 such that rotation of the locking tool (e.g., clockwise rotation) causes the nut 216 to rotate in the same direction (e.g., clockwise) and advance distally along the locking screw 202. The notched portions 218, 226 in the illustrated embodiment are configured such that rotation of the locking tool 224 in the opposite direction (e.g., counter-clockwise) allows the notched portion 226 of the tool 224 to disengage the notched portion 218 of the locking nut 216; that is, rotation of the locking tool in a direction that causes the locking tool to move proximally does not cause corresponding rotation of the nut.

In alternative embodiments, the distal end portion of the locking tool 224 can have various other configurations adapted to engage the nut 216 and produce rotation of the nut upon rotation of the locking tool for moving the nut distally, such as any of the tool configurations described herein. In some embodiments, the distal end portion of the locking tool 224 can be adapted to produce rotation of the nut 216 in both directions so as move the nut distally and proximally along the locking screw 202.

In operation, prior to implantation, the actuator member 222 is screwed onto the proximal end portion 206 of the actuator screw 202 and the locking nut 216 is rotated such that it is positioned at the proximal end of the screw. The frame 104 can then be placed in a radially collapsed state and the delivery assembly can be inserted into a patient. Once the prosthetic valve is at a desired implantation site, the frame 104 can be radially expanded as described herein.

To radially expand the frame 104, the support tube 220 is held firmly against the sleeve 212. The actuator member 222 is then pulled in a proximal direction through the support tube, such as by pulling on the proximal end of the actuator member or actuating a control knob on the handle that produces proximal movement of the actuator member. Because the support tube 220 is being held against the sleeve 212, which is connected to a proximal end of the frame 104 by the proximal valve connector 214, the proximal end of the frame is prevented from moving relative to the support tube. As such, movement of the actuator member 222 in a proximal direction causes movement of the actuator screw 202 in a proximal direction (because the actuator member is threaded onto the screw), thereby causing the frame 104 to foreshorten axially and expand radially. Alternatively, the frame 104 can be expanded by moving the support tube 220 distally while holding the actuator member 222 stationary or moving the support tube distally while moving the actuator member 222 proximally.

After the frame 104 is expanded to a desired radially expanded size, the frame can be locked at this radially expanded size as described herein. Locking the frame can be achieved by rotating the locking tool 224 in a clockwise direction causing the notched portion 226 of the locking tool to engage the notched portion 218 of the locking nut 216, thereby advancing the locking nut distally along the actuator screw 202. The locking tool 224 can be so rotated until the locking nut 216 abuts an internal shoulder at the distal end of the sleeve 212 and the locking nut 216 cannot advance distally any further (see FIG. 6). This will prevent the screw 202 from advancing distally relative to the sleeve 212 and radially compressing the frame 104. However, in the illustrated embodiment, the nut 216 and the screw 202 can still move proximally through the sleeve 212, thereby allowing additional expansion of the frame 104 either during implantation or later during a valve-in-valve procedure.

Once the frame 104 is locked in radially expanded state, the locking tool 224 can be rotated in a direction to move the locking tool proximally (e.g., in a counter-clockwise direction) to decouple the notched portion 226 from the notched portion 218 of the locking nut 216 and to unscrew the locking tool from the actuator screw 204. Additionally, the actuator member 222 can be rotated in a direction to unscrew the actuator member from the lower portion 206 of the actuator screw 202 (e.g., the actuator member 222 can be configured to disengage from the actuator screw when rotated counter-clockwise). Once the locking tool 224 and the actuator member 222 are unscrewed from the actuator screw 204, they can be removed from the patient along with the support tube 220, leaving the actuator screw and the sleeve 212 connected to the frame 104, as shown in FIG. 5C, with the frame 104 locked in a particular radially-expanded state.

In an alternative embodiment, the locking tool 224 can be formed without internal threads that engage the external threads of the actuator screw 202, which can allow the locking tool 224 to be slid distally and proximally through the sleeve 212 and along the actuator screw 202 to engage and disengage the nut 216.

In some embodiments, additional designs for expansion and locking mechanisms can be used instead of the design previously described. Details on expansion and locking mechanisms can be found, for example, in U.S. Patent Application Publication No. 2018/0153689.

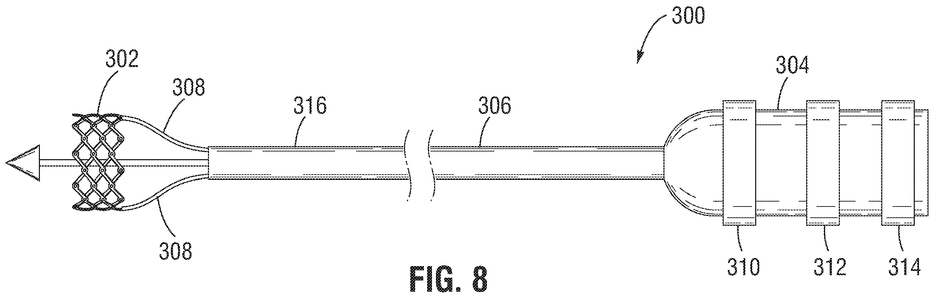

FIG. 8 illustrates a delivery apparatus 300, according to one embodiment, adapted to deliver a prosthetic heart valve 302, such as the illustrated prosthetic heart valve 10 or 100, described above. The prosthetic valve 302 can be releasably coupled to the delivery apparatus 300. It should be understood that the delivery apparatus 300 and other delivery apparatuses disclosed herein can be used to implant prosthetic devices other than prosthetic valves, such as stents or grafts.

The delivery apparatus 300 in the illustrated embodiment generally includes a handle 304, a first elongated shaft 306 (which comprises an outer shaft in the illustrated embodiment) extending distally from the handle 304, at least one actuator assembly 308 extending distally through the outer shaft 306. The at least one actuator assembly 308 can be configured to radially expand and/or radially collapse the prosthetic valve 302 when actuated.

Though the illustrated embodiment shows two actuator assemblies 308 for purposes of illustration, it should be understood that one actuator 308 can be provided for each actuator on the prosthetic valve. For example, three actuator assemblies 308 can be provided for a prosthetic valve having three actuators. In other embodiments, a greater or fewer number of actuator assemblies can be present.

In some embodiments, a distal end portion 316 of the shaft 306 can be sized to house the prosthetic valve in its radially compressed, delivery state during delivery of the prosthetic valve through the patient's vasculature. In this manner, the distal end portion 316 functions as a delivery sheath or capsule for the prosthetic valve during delivery.

The actuator assemblies 308 can be releasably coupled to the prosthetic valve 302. For example, in the illustrated embodiment, each actuator assembly 308 can be coupled to a respective actuator 200 of the prosthetic valve 302. Each actuator assembly 308 can comprise a support tube 220, an actuator member 222, and a locking tool 224. When actuated, the actuator assembly can transmit pushing and/or pulling forces to portions of the prosthetic valve to radially expand and collapse the prosthetic valve as previously described. The actuator assemblies 308 can be at least partially disposed radially within, and extend axially through, one or more lumens of the outer shaft 306. For example, the actuator assemblies 308 can extend through a central lumen of the shaft 306 or through separate respective lumens formed in the shaft 306.

The handle 302 of the delivery apparatus 300 can include one or more control mechanisms (e.g., knobs or other actuating mechanisms) for controlling different components of the delivery apparatus 300 in order to expand and/or deploy the prosthetic valve 10. For example, in the illustrated embodiment the handle 302 comprises first, second, and third knobs 310, 312, and 314.

The first knob 310 can be a rotatable knob configured to produce axial movement of the outer shaft 306 relative to the prosthetic valve 302 in the distal and/or proximal directions in order to deploy the prosthetic valve from the delivery sheath 316 once the prosthetic valve has been advanced to a location at or adjacent the desired implantation location with the patient's body. For example, rotation of the first knob 310 in a first direction (e.g., clockwise) can retract the sheath 316 proximally relative to the prosthetic valve 302 and rotation of the first knob 310 in a second direction (e.g., counter-clockwise) can advance the sheath 316 distally. In other embodiments, the first knob 310 can be actuated by sliding or moving the knob 310 axially, such as pulling and/or pushing the knob. In other embodiments, actuation of the first knob 310 (rotation or sliding movement of the knob 310) can produce axial movement of the actuator assemblies 308 (and therefore the prosthetic valve 302) relative to the delivery sheath 316 to advance the prosthetic valve distally from the sheath 316.

The second knob 312 can be a rotatable knob configured to produce radial expansion and/or contraction of the prosthetic valve 302. For example, rotation of the second knob 312 can move the actuator member 222 and the support tube 220 axially relative to one another. Rotation of the second knob 312 in a first direction (e.g., clockwise) can radially expand the prosthetic valve 302 and rotation of the second knob 312 in a second direction (e.g., counter-clockwise) can radially collapse the prosthetic valve 302. **In** other embodiments, the second knob 312 can be actuated by sliding or moving the knob 312 axially, such as pulling and/or pushing the knob.

The third knob 314 can be a rotatable knob configured to retain the prosthetic heart valve 302 in its expanded configuration. For example, the third knob 314 can be operatively connected to a proximal end portion of the locking tool 224 of each actuator assembly 308. Rotation of the third knob in a first direction (e.g., clockwise) can rotate each locking tool 224 to advance the locking nuts 216 to their distal positions to resist radial compression of the frame of the prosthetic valve, as described above. Rotation of the knob 314 in the opposite direction (e.g., counterclockwise) can rotate each locking tool 224 in the opposite direction to decouple each locking tool 224 from the respective nut 216 and remove the locking tool 224 from the respective actuator screw 202. In other embodiments, the third knob 314 can be actuated by sliding or moving the third knob 314 axially, such as pulling and/or pushing the knob.

Although not shown, the handle 304 can include a fourth rotatable knob operative connected to a proximal end portion of each actuator member 222. The fourth knob can be configured to rotate each actuator member 222, upon rotation of the knob, to unscrew each actuator member 222 from the proximal portion 206 of a respective actuator 202. As described above, once the locking tools 224 and the actuator members 222 are unscrewed form the actuator screws 204, they can be removed from the patient along with the support tubes 220.

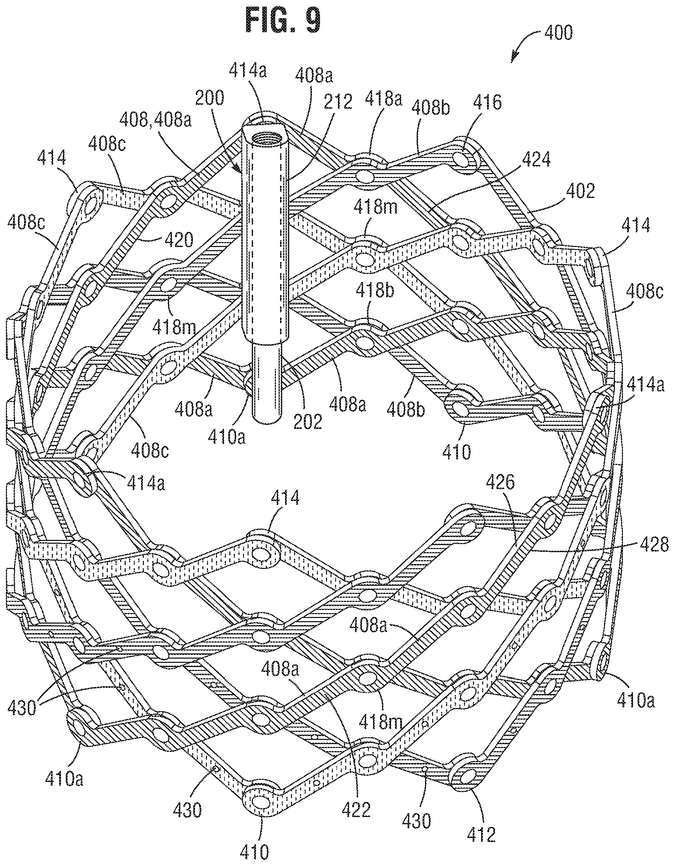

FIG. 9 illustrates another exemplary embodiment of a prosthetic valve 400 comprising frame 402. The prosthetic valve 400 can include a valvular structure (e.g., valvular structure 18) and inner and/or outer skirts, as previously described, although these components are omitted for purposes of illustration.

The frame 402 can include a plurality of interconnected struts 408 arranged in a lattice-type pattern and forming a plurality of distal apices 410 at the inflow end 412 of the frame 402 and a plurality of proximal apices 414 at the outflow end 416 of the frame. Each strut 408 can be coupled to one or more other struts 408 at a plurality of junctions 418 forming a plurality of cells 420. Each distal and proximal apex 410, 414 is also a junction 418.

The struts 408 are arranged in different sets of struts, namely, a first set of first struts 408a, a second set of second struts 408b, and a third set of third struts 408c. For purposes of illustration, the first, second, and third struts 408a, 408b, 408c include different fill patterns in FIG. 9. In alternative embodiments, the frame can be formed from a greater or fewer number of sets of struts, such as two sets of struts, or four sets of struts.

In the illustrated embodiment, each strut of a particular set is pivotably coupled to another strut of the same set at a distal apex 410, another strut of the same set at a proximal apex 414, and another strut of the same set at a junction 418 between the distal and proximal apices 410, 414 (desirably at a middle junction 418m at the midsections of the two overlapping struts equidistant from the distal and proximal apices). Thus, for example, each first strut 408a of the first set of struts is pivotably coupled to another first strut 408a of the first set at a distal apex 410, another first strut 408a of the first set at a proximal apex 414, and another first strut 408a of the first set at a middle junction 418m at the midsections of the two overlapping first struts wherein the middle junction 418m is equidistant from the distal and proximal apices.

Except where each strut 408a, 408b, 408c is pivotably coupled to a strut of the same set at a distal apex 410, a proximal apex 414, and a middle junction 418m, the strut can be pivotably coupled to a strut of a different set at junctions between the distal apex 410 and the middle junction 418m and at junctions between the proximal apex 414 and the middle junction 418m. For example, a first strut 408a can be pivotably coupled to a second strut 408b at a junction 418a, which is the junction along those struts closest to the proximal apices 414 at the outflow end 416. A first strut 408a can be pivotably coupled to a second strut 408b at a junction 418b, which is the junction along those struts closest to the distal apices 410 at the inflow end 412.

In alternative embodiments, struts of one set can be pivotably coupled to struts of another set at the distal apices 410, the proximal apices 414, and/or the middle junctions 418m.

One or more actuators (e.g., actuators 200) can be coupled to the frame 402 at the distal and proximal apices 410, 414. FIG. 9 schematically shows an actuator 200 coupled to the frame 402 at a distal apex 410a formed by two first struts 408a and at a proximal apex 414a formed by two first struts 408a. For example, an actuator screw 202 of the actuator 200 can be coupled to the distal apex 410a via a distal valve connector 210 (FIG. 6) and a sleeve 212 of the actuator 200 can be coupled to the proximal apex 414a via a proximal valve connector 214 (FIG. 6). Additional actuators 200 can be coupled to the frame 402 in the same manner, with each actuator coupled to a pair of a distal apex 410a formed by two first struts 408a and at a proximal apex 414a formed by two first struts 408a. In particular embodiments, the prosthetic valve 400 can include three actuators 200, similar to the embodiment shown in FIG. 5C.

The actuators 200 can be releasably coupled to respective actuator assemblies of a delivery apparatus (e.g., actuator assemblies 308 of delivery apparatus 300), which transfer expansion and compression forces to the actuators 200 to radial expand and compress the prosthetic valve 400, as discussed above. In the depicted embodiment, the inflow end 412 is the distal end of the frame that includes the distal apices 410 and the outflow end 416 is the proximal end that includes the proximal apices 412. The actuators 200 can be coupled to respective actuator assemblies of a delivery apparatus at the outflow end 416 of the frame. This orientation is suitable for delivering and implanting the prosthetic valve within the native aortic valve in a transfemoral, retrograde delivery approach. Thus, in the delivery configuration of the prosthetic valve, the outflow end 416 is the proximal-most end of the prosthetic valve. In other embodiments, the inflow end 412 can be coupled to the delivery apparatus, depending on the particular native valve being replaced and the delivery technique that is used (e.g., trans-septal, transapical, etc.). For example, the inflow end 412 can be coupled to the delivery apparatus (and therefore is the proximal-most end of the prosthetic valve in the delivery configuration) when delivering the prosthetic valve to the native mitral valve via a trans-septal delivery approach.

By virtue of the actuators 200 being coupled to the apices 410a, 414a of the first struts 408a, the first struts 408a will experience higher stresses than the distal and proximal apices formed by the second struts 408b and the third struts 404c due to the expansion forces applied by the actuators to the apices 410a, 414a during valve expansion and the retaining forces applied by the actuators to the apices 410a, 414a to resist radial compression of the frame 402 against outside forces (e.g., the force of the surrounding native annulus or tissue) once the prosthetic valve is expanded to its function size. In contrast, these forces are not directly applied to the apices 410, 414 formed by the second struts 408b and the third struts 408c. As such, the first struts 408a can be configured to have a greater resistance against deformation caused by axial directed forces applied to the apices 410a, 414a than the second struts 408b and/or the third struts 408c. The first struts 408a therefore can be referred to as "reinforced struts" and the second struts 408b and the third struts 408c can be referred to as "free struts". The reinforced struts 408a can have a greater resistance than the free struts 408b, 408c against buckling and/or bending relative to a radially extending transverse axis that is perpendicular to a longitudinal axis of the prosthetic valve 400 caused by axially directed forces applied to the frame.

In particular embodiments, a cross-sectional profile of the first struts 408a (taken in a plane perpendicular to the length of the first struts) has a dimension that is greater than a corresponding dimension of a cross-sectional profile of the second struts 408b, 408c to increase the stiffness and rigidity of the first struts, thereby providing the first struts with a greater resistance against deformation from axially directed forces applied to the frame.

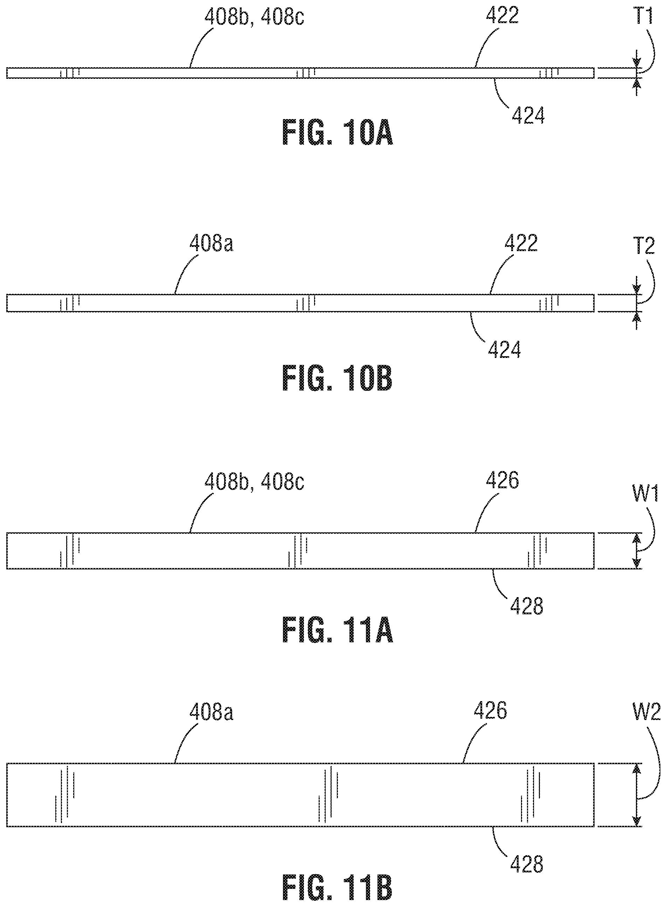

For example, in particular embodiments, the first struts 408a can have a greater thickness than that of the second struts 408b and/or the third struts 408c to better resist the forces applied to apices 410a, 414a. For example, as shown in FIGS. 10A and 10B, the second and third struts 408b, 408c can have a first thickness T1 and the first struts 408a can have thickness T2, wherein T1 is less than T2. The thicknesses T1 and T2 are measured between a radially facing outer surface 422 of the strut and a radially facing inner surface 424 of the strut. In some embodiments, the second and third struts 408b, 408c, respectively, can have different thicknesses, but desirably are both less than the thickness T2 of the first struts.

In particular embodiments, the thickness T1 of the second and third struts 408b, 408c can be about 0.1 mm to about 0.5 mm, and more desirably about 0.3 mm to about 0.35 mm, with 0.3 mm being a particular example. The thickness T2 of the first struts 408a can be about 0.2 mm to about 0.8 mm, and more desirably about 0.4 mm to about 0.5 mm, with 0.45 mm being a particular example.

The first struts 408a can have a constant thickness T2 along their entire longitudinal length, or they may have a varying thickness. Similarly, the second struts 408b and the third struts 408c can have a constant thickness T1 along their entire length, or they may have a varying thickness. Where the struts 408a, 408b, 408c have a variable thickness, a minimum thickness T2 of the first struts 408a need not be greater than a maximum thickness T1 of the second and third struts 408b, 408c. However, the average thickness T2 along the length of the first struts 408a desirably is greater than the average thickness T1 along the length of the second and third struts 408b, 408c.

In some embodiments, the first struts 408a can have a greater width than that of the second struts 408b and/or the third struts 408c to better resist the forces applied to apices 410a, 414a. For example, as shown in FIGS. 11A and 11B, the second and third struts 408b, 408c can have a first width W1 and the first struts 408a can have width W2, wherein W1 is less than W2. The widths W1 and W2 are measured between a first longitudinal edge 426 of the strut facing the proximal end 416 of the frame 402 and a second longitudinal edge 428 of the strut facing the distal end 412 of the frame 402.

In particular embodiments, the width W1 of the second and third struts 408b, 408c can be about 0.7 mm to about 0.9 mm, and more desirably about 0.74 mm to about 0.8 mm, with 0.74 mm being a particular example. The width W2 of the first struts 408a can be about 0.7 mm to about 1.2 mm, and more desirably about 0.74 mm to about 0.9 mm, with 0.85 mm being a particular example.

The first struts 408a can have a constant width W2 along their entire longitudinal length, or they may have a varying width. Similarly, the second struts 408b and the third struts 408c can have a constant width W1 along their entire length, or they may have a varying width. Where the struts 408a, 408b, 408c have a variable width, a minimum width W2 of the first struts 408a need not be greater than a maximum width W1 of the second and third struts 408b, 408c. However, the average width W2 along the length of the first struts 408a desirably is greater than the average width W1 along the length of the second and third struts 408b, 408c.

In some embodiments, the first struts 408a can have a greater thickness T2 and a greater width W2 than the second struts 408b and/or the third struts 408c.

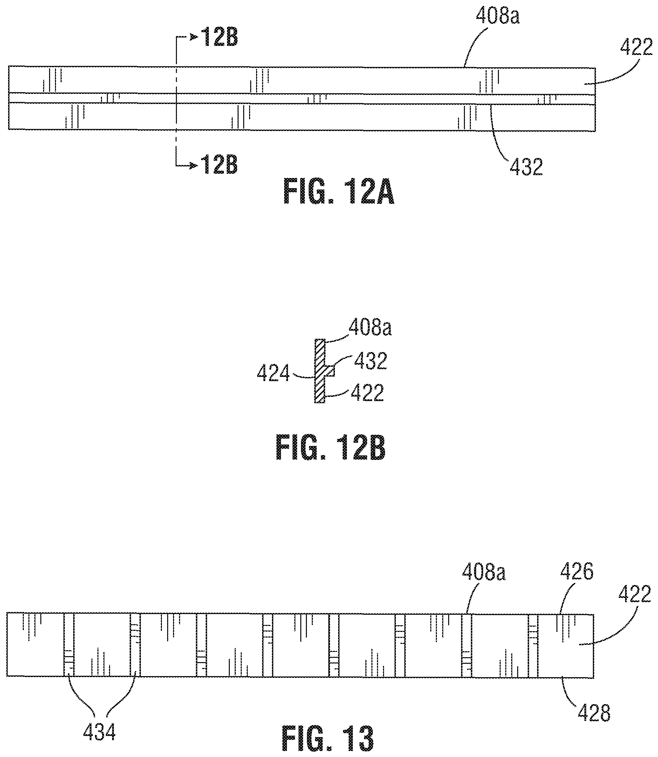

In alternative embodiments, the first struts 408a can have other geometries that reinforce those struts and increase the resistance against deformation. For example, as shown in FIGS. 12A and 12B, the first struts 408a can be reinforced with one or more ribs or protrusions 432 that extend lengthwise of the struts. The one or more ribs 432 can extend from the radially facing outer surface 422 of the strut (as shown in FIGS. 12A-12B), the radially facing inner surface 424 of the strut, or both the outer and inner surfaces 422, 424. In another embodiment, as shown in FIG, 13, the first struts 408a can be reinforced with a plurality of transverse ribs or protrusions 434 extending widthwise of the struts and spaced apart from each other along the length of the struts. The ribs 434 can extend from the radially facing outer surface 422 of the strut (as shown in FIG. 13), the radially facing inner surface 424 of the strut, or both the outer and inner surfaces 422, 424. In still other embodiments, the first struts 408a can be reinforced with a combination of longitudinal ribs 432 and transverse ribs 434.

In some embodiments, in lieu of or in addition to forming the struts with different dimensions (different thickness and/or widths) or geometries, the first struts 408a can be made of a stronger or more rigid or stiffer material than the second struts 408b and/or the third struts 408c so that the first struts 408a can better resist forces applied to those struts. For example, the first struts 408a can be made of a material that has a greater modulus of elasticity than the material(s) used to make the second and third struts 408b, 408c. In particular embodiments, for example, the first struts 408a can be made of stainless steel or a cobalt chromium alloy, while the second struts 408b and/or the third struts 408c can be made of a nickel titanium alloy (e.g., Nitinol).

Advantageously, providing struts 408b, 408c with different geometries and/or dimensions than struts 408a (e.g., struts 408b, 408c that are thinner than struts 408a) allows the frame 402 to be radially crimped to a more compact, smaller diameter in the delivery configuration (such as shown in FIG. 2A and 3). This can reduce the overall cross-sectional profile of the prosthetic valve and the delivery apparatus to facilitate passage of the prosthetic valve and the delivery apparatus through the vasculature of the patient.

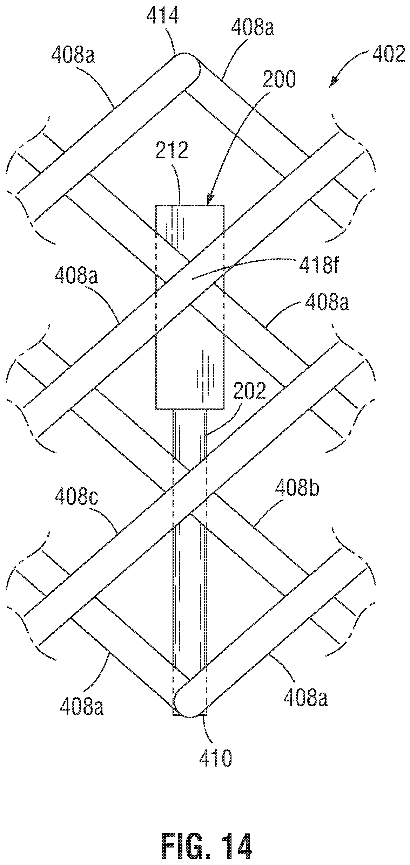

In alternative embodiments, one or both of the two components of the actuators (e.g., the inner member 202 and the outer member 212) can be coupled to junctions of the frame other than at the proximal and distal apices by replacing a strut of one set with a strut of another set. FIG. 14, for example, shows a portion of a frame 402 in which the struts of the first, second, and third sets are arranged in a different configuration, according to one embodiment. In FIG. 14, a strut 408b of the second set and a strut 408c of the third set are both replaced with a strut 408a of the first set. The two additional struts 408a form a junction 418f spaced from a proximal apex 414 by one frame cell toward the inflow end of the frame. The outer member 212 of an actuator 200 can be pivotably coupled to the struts 408a at junction 418f and the inner member 202 of the actuator 200 can be pivotably coupled to the struts 408a forming the distal apex 410a. Other sections of the frame 402 can be similarly configured with the arrangement of struts shown in FIG. 14 and can include an actuator 200 coupled to the frame as shown in FIG. 14. In some embodiments, one or both of the two struts 408a forming the proximal apex 414 can be replaced with a strut 408b or a strut 408c.

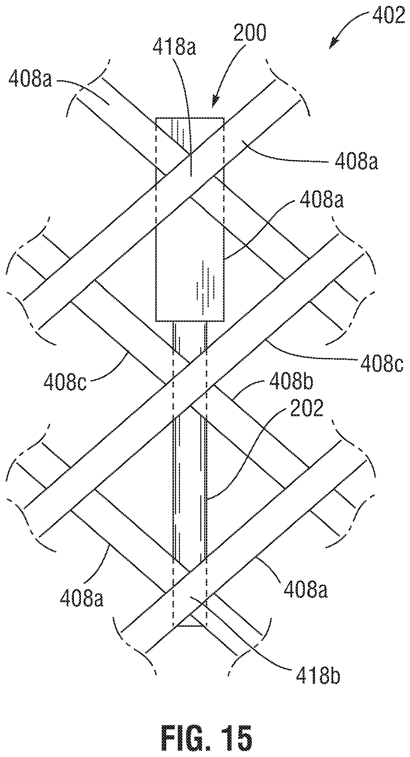

FIG. 15 shows a portion of a frame 402, according to another embodiment. The portion of the frame shown in FIG. 15 comprises the sections of struts circumferentially between a first pair of proximal and distal apices 414, 410 and a second pair of proximal and distal apices 414, 410. In FIG. 15, two struts 408a form junction 418a and two struts 408a form junction 418b. Thus, this portion of the frame need not include any struts 408b of the second set. The outer member 212 of an actuator 200 can be pivotably coupled to the struts 408a at junction 418a and the inner member 202 of the actuator 200 can be pivotably coupled to the struts 408a forming junction 418b. Other sections of the frame 402 can be similarly configured with the arrangement of struts shown in FIG. 15 and can include an actuator 200 coupled to the frame as shown in FIG. 15. In some embodiments, the entire frame 402 can be comprised of only struts 408a and 408c.

Various other types of actuators can be coupled to the frame 402 at the distal and proximal apices 410a, 414a, including any actuators disclosed in U.S. Publication No. 2018/0153689 or U.S. Application No. 63/013,912. For example, in one embodiment, one or more actuators 50 (FIG. 1) can be coupled to the frame 402 with the sleeve 54 of each actuator 50 being coupled to a proximal apex 414a and the nut 56 of each actuator being coupled to a distal apex 410a.

As noted above, the prosthetic valve 400 can include a valvular structure (e.g., valvular structure 18 comprising a plurality of leaflets 20) and inner and/or outer skirts, as previously described. The commissures of the leaflets 20 can be mounted to the actuators 200 in a similar manner as shown in FIG. 1 where the commissures are mounted to actuators 50. In alternative embodiments, the commissures of the leaflets 20 can be mounted to separate commissure posts that are mounted to the second struts 408b and/or the third struts 408c. For example, separate commissure posts can be mounted to the proximal apices 414 formed by the second struts 408b and/or the third struts 408c.

The inner skirt and/or the outer skirt can be mounted to the frame 402 using sutures that extend through the skirt and openings 430 in the second struts 408b and/or the third struts 408c. In the illustrated embodiment, the openings 430 are formed in strut segments of the second and third struts 408b, 408c between the distal apices 410 and the middle junctions 418m of the second and third struts. However, if desired, openings 430 can be formed in each strut segment between adjacent junctions of the second and third struts. Also, although one opening 430 is shown in each strut segment, more than one opening 430 can be provided in each strut segment between adjacent junctions. Moreover, in alternative embodiments, openings 430 can be provided in the first struts 408a for attaching the inner and/or outer skirts. Further details regarding the attachment of inner and outer skirts to the frame are disclosed in U.S. Provisional Application No. 62/854,702. Advantageously, utilizing openings in the struts simplifies the assembly process for the prosthetic valve.

### General Considerations

It should be understood that the disclosed embodiments can be adapted for delivering and implanting prosthetic devices in any of the native annuluses of the heart (e.g., the aortic, pulmonary, mitral, and tricuspid annuluses), and can be used with any of various delivery devices for delivering the prosthetic valve using any of various delivery approaches (e.g., retrograde, antegrade, transseptal, transventricular, transatrial, etc.).

For purposes of this description, certain aspects, advantages, and novel features of the embodiments of this disclosure are described herein. The disclosed methods, apparatus, and systems should not be construed as being limiting in any way. Instead, the present disclosure is directed toward all novel and nonobvious features and aspects of the various disclosed embodiments, alone and in various combinations and sub-combinations with one another. The methods, apparatus, and systems are not limited to any specific aspect or feature or combination thereof, nor do the disclosed embodiments require that any one or more specific advantages be present or problems be solved. The technologies from any example can be combined with the technologies described in any one or more of the other examples. In view of the many possible embodiments to which the principles of the disclosed technology may be applied, it should be recognized that the illustrated embodiments are only preferred examples and should not be taken as limiting the scope of the disclosed technology.

Although the operations of some of the disclosed embodiments are described in a particular, sequential order for convenient presentation, it should be understood that this manner of description encompasses rearrangement, unless a particular ordering is required by specific language set forth herein. For example, operations described sequentially may in some cases be rearranged or performed concurrently. Moreover, for the sake of simplicity, the attached figures may not show the various ways in which the disclosed methods can be used in conjunction with other methods. Additionally, the description sometimes uses terms like "provide" or "achieve" to describe the disclosed methods. These terms are high-level abstractions of the actual operations that are performed. The actual operations that correspond to these terms may vary depending on the particular implementation and are readily discernible by one of ordinary skill in the art.

As used in this application and in the claims, the singular forms "a," "an," and "the" include the plural forms unless the context clearly dictates otherwise. Additionally, the term "includes" means "comprises." Further, the terms "coupled" and "connected" generally mean electrically, electromagnetically, and/or physically (e.g., mechanically or chemically) coupled or linked and does not exclude the presence of intermediate elements between the coupled or associated items absent specific contrary language.

Directions and other relative references (e.g., inner, outer, upper, lower, etc.) may be used to facilitate discussion of the drawings and principles herein, but are not intended to be limiting. For example, certain terms may be used such as "inside," "outside,", "top," "down," "interior," "exterior," and the like. Such terms are used, where applicable, to provide some clarity of description when dealing with relative relationships, particularly with respect to the illustrated embodiments. Such terms are not, however, intended to imply absolute relationships, positions, and/or orientations. For example, with respect to an object, an "upper" part can become a "lower" part simply by turning the object over. Nevertheless, it is still the same part and the object remains the same. As used herein, "and/or" means "and" or "or", as well as "and" and "or".

In view of the many possible embodiments to which the principles of the disclosed invention may be applied, it should be recognized that the illustrated embodiments are only preferred examples of the invention and should not be taken as limiting the scope of the invention. Rather, the scope of the invention is defined by the following claims.

## Claims

1. An implantable prosthetic device (400), comprising:
a frame (402) being radially expandable and compressible between a radially compressed state and a radially expanded state, the frame comprising a first set of first struts (408a) and a second set of second struts (408b), the frame having a distal end and a proximal end;
wherein the first struts (408a) are pivotably connected to each other at a plurality of distal and proximal apices (410, 414) at the distal and proximal ends of the frame, respectively;
wherein the second struts (408b) are pivotably connected to each other at a plurality of distal and proximal apices (410, 414), at the distal and proximal ends of the frame, respectively;
wherein the first struts are pivotably connected to the second struts at junctions (418) between the distal and proximal ends of the frame (402);
wherein the first struts (408a) have a first thickness (T2), which is measured between a radially facing inner surface (424) and a radially facing outer surface (422) of each first strut;
wherein the second struts have a second thickness (T1), which is measured between a radially facing inner surface (424) and a radially facing outer surface (422) of each second strut;
wherein the first thickness (T2) is greater than the second thickness (T1); and
one or more actuators (200) coupled only to one or more corresponding pairs of a distal apex (410) and a proximal apex (414) formed by the first struts (408a), wherein the one or more actuators (200) are configured to apply axially directed forces to the frame (402) to radially expand the frame from the radially compressed state to the radially expanded state.

2. The implantable prosthetic device of claim 1, wherein the one or more actuators (200) are configured to retain the frame in the radially expanded state.

3. The implantable prosthetic device of any previous claim, wherein the one or more actuators (200) comprise three actuators, each of which is coupled to a corresponding pair of a distal apex (410) and a proximal apex (414) formed by the first struts (408a).

4. The implantable prosthetic device of any previous claim, wherein the first struts (408a) are made of a first material, the second struts (408b) are made of a second material, and the first material is more rigid than the second material.

5. The implantable prosthetic device of any previous claim, wherein:
the first struts (408a) are pivotably connected to each other at middle junctions (418m) located at midsections of the first struts (408a); and
the second struts (408b) are pivotably connected to each other at middle junctions (418m) located at midsections of the second struts (408b).

6. The implantable prosthetic device of any previous claim, wherein:
the frame (402) comprises a third set of third struts (408c), wherein the third struts are pivotably connected to each other at a plurality of distal and proximal apices (410, 414) at the distal and proximal ends of the frame, respectively;
the third struts are pivotably connected to the first and second struts at junctions (418) between the distal and proximal ends of the frame;
the third struts have a third thickness (T1), which is measured between a radially facing inner surface and a radially facing outer surface of each third strut; and
the first thickness (T2) is greater than the third thickness (T1).

7. The implantable prosthetic device of claim 6, wherein the second thickness (T1) and the third thickness (T1) are the same.

8. The implantable prosthetic device of any previous claim, wherein the first struts (408a) have a greater resistance against deformation from axially directed forces applied to the frame than the second struts (408b).

9. The implantable prosthetic device of any previous claim, further comprising a plurality of leaflets (20) disposed in the frame and configured to regulate a flow of blood through the frame in one direction.

10. The implantable prosthetic device of any previous claim, further comprising a fabric skirt mounted to the frame with sutures extending through openings in the second struts.

11. The implantable prosthetic device of any of claims 1 to 10, wherein the first struts comprise reinforcing ribs.

12. The implantable prosthetic device of claim 11, wherein each first strut comprises one or more ribs (432) that extend lengthwise of the first struts (408a).

13. The implantable prosthetic device of claim 11, wherein each first strut comprises a plurality of spaced-apart transverse ribs (434) extending widthwise of the first struts (408a).

14. The implantable prosthetic device of any of claims 1 to 13, wherein each of one or more actuators (200) comprises first and second members (212, 202) axially moveable relative to each other, wherein the first member (212) is pivotably coupled to a distal apex (410) and the second member (202) is pivotably coupled to a proximal apex (414) formed by the first struts (408a).

15. The implantable prosthetic device of claim 14, wherein the first member is an outer member (212) and the second member is an inner member (202) that is partially received within the outer member (212).

## Patentansprüche

1. Implantierbare prothetische Vorrichtung (400), umfassend:
einen Rahmen (402), der radial zwischen einem radial komprimierten Zustand und einem radial expandierten Zustand expandierbar und komprimierbar ist, wobei der Rahmen eine erste Gruppe von ersten Streben (408a) und eine zweite Gruppe von zweiten Streben (408b) umfasst, wobei der Rahmen ein distales Ende und ein proximales Ende aufweist;
wobei die ersten Streben (408a) entsprechend an einer Vielzahl von distalen und proximalen Spitzen (410, 414) an dem distalen und proximalen Ende des Rahmens schwenkbar miteinander verbunden sind;
wobei die zweiten Streben (408b) entsprechend an einer Vielzahl von distalen und proximalen Spitzen (410, 414) an dem distalen und dem proximalen Ende des Rahmens schwenkbar miteinander verbunden sind;
wobei die ersten Streben schwenkbar mit den zweiten Streben an Verbindungsstellen (418) zwischen dem distalen und dem proximalen Ende des Rahmens (402) verbunden sind;
wobei die ersten Streben (408a) eine erste Dicke (T2) aufweisen, die zwischen einer radial nach innen weisenden Oberfläche (424) und einer radial nach außen weisenden Oberfläche (422) jeder ersten Strebe gemessen wird;
wobei die zweiten Streben eine zweite Dicke (T1) aufweisen, die zwischen einer radial nach innen weisenden Oberfläche (424) und einer radial nach außen weisenden Oberfläche (422) jeder zweiten Strebe gemessen wird;
wobei die erste Dicke (T2) größer ist als die zweite Dicke (T1); und
einen oder mehrere Aktuatoren (200), die nur mit jeweils einem oder mehreren zugeordneten Paaren einer distalen Spitze (410) und einer proximalen Spitze (414), die von den ersten Streben (408a) gebildet werden, gekoppelt sind, wobei der eine oder die mehreren Aktuatoren (200) dazu konfiguriert sind, axial gerichtete Kräfte auf den Rahmen (402) auszuüben, um den Rahmen vom radial komprimierten Zustand in den radial expandierten Zustand radial zu expandieren.

2. Implantierbare prothetische Vorrichtung gemäß Anspruch 1, wobei der eine oder die mehreren Aktuatoren (200) dazu konfiguriert sind, den Rahmen im radial expandierten Zustand zu halten.

3. Implantierbare prothetische Vorrichtung gemäß einem der vorangegangenen Ansprüche, wobei der eine oder die mehreren Aktuatoren (200) drei Aktuatoren umfassen, wovon jeder mit einem zugeordneten Paar von einer distalen Spitze (410) und einer proximalen Spitze (414), die von den ersten Streben (408a) gebildet werden, gekoppelt ist.

4. Implantierbare prothetische Vorrichtung gemäß einem der vorangegangenen Ansprüche, wobei die ersten Streben (408a) aus einem ersten Material gefertigt sind, die zweiten Streben (408b) aus einem zweiten Material gefertigt sind, und wobei das erste Material steifer ist als das zweite Material.

5. Implantierbare prothetische Vorrichtung gemäß einem der vorangegangenen Ansprüche, wobei:
die ersten Streben (408a) an mittleren Verbindungsstellen (418m), die sich in Mittelabschnitten der ersten Streben befinden, schwenkbar miteinander verbunden sind, und
die zweiten Streben (408b) an mittleren Verbindungsstellen (418m), die sich in Mittelabschnitten der zweiten Streben (408b) befinden, schwenkbar miteinander verbunden sind.

6. Implantierbare prothetische Vorrichtung gemäß einem der vorangegangenen Ansprüche, wobei:
der Rahmen (402) eine dritte Gruppe von dritten Streben (408c) umfasst, wobei die dritten Streben entsprechend an einer Vielzahl von distalen und proximalen Spitzen (410, 414) an dem distalen und proximalen Ende des Rahmens schwenkbar miteinander verbunden sind,
die dritten Streben an Verbindungsstellen (418) zwischen dem distalen und dem proximalen Ende des Rahmens schwenkbar mit den ersten und zweiten Streben verbunden sind,
die dritten Streben eine dritte Dicke (T1) aufweisen, die zwischen einer radial nach innen weisenden Oberfläche und einer radial nach außen weisenden Oberfläche jeder dritten Strebe gemessen wird, und
die erste Dicke (T2) größer ist als die dritte Dicke (T1).

7. Implantierbare prothetische Vorrichtung gemäß Anspruch 6, wobei die zweite Dicke (T1) und die dritte Dicke (T1) gleich sind.

8. Implantierbare prothetische Vorrichtung gemäß einem der vorangegangenen Ansprüche, wobei die ersten Streben (408a) eine höhere Widerstandsfähigkeit gegen Verformung durch axial gerichtete Kräfte, die auf den Rahmen aufgebracht werden, aufweisen als die zweiten Streben (408b).

9. Implantierbare prothetische Vorrichtung gemäß einem der vorangegangenen Ansprüche, darüber hinaus umfassend eine Vielzahl von Segeln (20), die im Rahmen angeordnet sind und dazu konfiguriert sind, einen Blutfluss durch den Rahmen in eine Richtung zu regulieren.

10. Implantierbare prothetische Vorrichtung gemäß einem der vorangegangenen Ansprüche, darüber hinaus umfassend eine Gewebeschürze, die mit Nähten, die durch Öffnungen in den zweiten Streben verlaufen, am Rahmen befestigt ist.

11. Implantierbare prothetische Vorrichtung gemäß einem der Ansprüche 1 bis 10, wobei die ersten Streben Verstärkungsrippen umfassen.

12. Implantierbare prothetische Vorrichtung gemäß Anspruch 11, wobei jede erste Strebe eine oder mehrere Rippen (432) umfasst, die sich in Längsrichtung der ersten Streben (408a) erstrecken.

13. Implantierbare prothetische Vorrichtung gemäß Anspruch 11, wobei jede erste Strebe mehrere beabstandete Querrippen (434) umfasst, die sich in Breitenrichtung der ersten Streben (408a) erstrecken.

14. Implantierbare prothetische Vorrichtung gemäß einem der Ansprüche 1 bis 13, wobei jeder des einen oder der mehreren Aktuatoren (200) ein erstes und ein zweites Element (212, 202) umfasst, die relativ zueinander axial beweglich sind, wobei das erste Element (212) schwenkbar mit einer distalen Spitze (410) gekoppelt ist und das zweite Element (202) schwenkbar mit einer proximalen Spitze (414), die von den ersten Streben (408a) gebildet wird, gekoppelt ist.

15. Implantierbare prothetische Vorrichtung gemäß Anspruch 14, wobei das erste Element ein äußeres Element (212) ist und das zweite Element ein inneres Element (202) ist, das teilweise in dem äußeren Element (212) aufgenommen ist.

## Revendications

1. Dispositif prothétique implantable (400), comprenant :
un cadre (402) déployable et compressible radialement entre un état radialement comprimé et un état radialement déployé, le cadre comprenant un premier ensemble de premières entretoises (408a) et un deuxième ensemble de deuxièmes entretoises (408b), le cadre présentant une extrémité distale et une extrémité proximale ;
les premières entretoises (408a) étant reliées de manière pivotante les unes aux autres au niveau d'une pluralité de sommets (410, 414) distaux et proximaux respectivement au niveau des extrémités distale et proximale du cadre ;
les deuxièmes entretoises (408b) étant reliées de manière pivotante les unes aux autres au niveau d'une pluralité de sommets (410, 414) distaux et proximaux respectivement au niveau des extrémités distale et proximale du cadre ;
les premières entretoises étant reliées de manière pivotante aux deuxièmes entretoises au niveau de jonctions (418) entre les extrémités distale et proximale du cadre (402) ;
les premières entretoises (408a) présentant une première épaisseur (T2), qui est mesurée entre une surface interne (424) orientée radialement et une surface externe (422) orientée radialement de chaque première entretoise ;
les deuxièmes entretoises présentant une deuxième épaisseur (T1), qui est mesurée entre une surface interne (424) orientée radialement et une surface externe (422) orientée radialement de chaque deuxième entretoise ;
la première épaisseur (T2) étant supérieure à la deuxième épaisseur (T1) ;
et
un ou plusieurs actionneurs (200) accouplés uniquement à une ou plusieurs paires correspondantes d'un sommet distal (410) et d'un sommet proximal (414) formés par les premières entretoises (408a), ledit un ou lesdits plusieurs actionneurs (200) étant conçus pour appliquer des forces dirigées axialement au cadre (402) afin de déployer radialement le cadre à partir de l'état radialement comprimé vers l'état radialement déployé.

2. Dispositif prothétique implantable selon la revendication 1, ledit un ou lesdits plusieurs actionneurs (200) étant conçus pour retenir le cadre dans l'état radialement déployé.

3. Dispositif prothétique implantable selon l'une quelconque revendication précédente, ledit un ou lesdits plusieurs actionneurs (200) comprenant trois actionneurs, dont chacun est accouplé à une paire correspondante d'un sommet distal (410) et d'un sommet proximal (414) formés par les premières entretoises (408a).

4. Dispositif prothétique implantable selon l'une quelconque revendication précédente, les premières entretoises (408a) étant constituées d'un premier matériau, les deuxièmes entretoises (408b) étant constituées d'un second matériau et le premier matériau étant plus rigide que le second matériau.

5. Dispositif prothétique implantable selon l'une quelconque revendication précédente :
les premières entretoises (408a) étant reliées de manière pivotante les unes aux autres au niveau de jonctions centrales (418m) situées au niveau de sections centrales des premières entretoises (408a) ; et
les deuxièmes entretoises (408b) étant reliées de manière pivotante les unes aux autres au niveau de jonctions centrales (418m) situées au niveau de sections centrales des deuxièmes entretoises (408b).

6. Dispositif prothétique implantable selon l'une quelconque revendication précédente :
le cadre (402) comprenant un troisième ensemble de troisièmes entretoises (408c), les troisièmes entretoises étant reliées de manière pivotante les unes aux autres au niveau d'une pluralité de sommets (410, 414) distaux et proximaux respectivement au niveau des extrémités distale et proximale du cadre ;
les troisièmes entretoises étant reliées de manière pivotante aux premières et deuxièmes entretoises au niveau de jonctions (418) entre les extrémités distale et proximale du cadre ;
les troisièmes entretoises présentant une troisième épaisseur (T1), qui est mesurée entre une surface interne orientée radialement et une surface externe orientée radialement de chaque troisième entretoise ; et
la première épaisseur (T2) étant supérieure à la troisième épaisseur (T1).

7. Dispositif prothétique implantable selon la revendication 6, la deuxième épaisseur (T1) et la troisième épaisseur (T1) étant les mêmes.

8. Dispositif prothétique implantable selon l'une quelconque revendication précédente, les premières entretoises (408a) présentant une plus grande résistance contre la déformation des forces dirigées axialement appliquées au cadre que celle des deuxièmes entretoises (408b).

9. Dispositif prothétique implantable selon l'une quelconque revendication précédente, comprenant en outre une pluralité de feuillets (20) disposés dans le cadre et conçus pour réguler un écoulement de sang à travers le cadre dans un sens.

10. Dispositif prothétique implantable selon l'une quelconque revendication précédente, comprenant en outre une collerette en tissu montée sur le cadre avec des sutures s'étendant à travers des ouvertures dans les deuxièmes entretoises.

11. Dispositif prothétique implantable selon l'une quelconque des revendications 1 à 10, les premières entretoises comprenant des nervures de renfort.

12. Dispositif prothétique implantable selon la revendication 11, chaque première entretoise comprenant une ou plusieurs nervures (432) qui s'étendent dans le sens de la longueur des premières entretoises (408a).

13. Dispositif prothétique implantable selon la revendication 11, chaque première entretoise comprenant une pluralité de nervures transversales (434) écartées s'étendant dans le sens de la largeur des premières entretoises (408a).

14. Dispositif prothétique implantable selon l'une quelconque des revendications 1 à 13, chaque actionneur parmi un ou plusieurs actionneurs (200) comprenant des premier et second éléments (212, 202) mobiles axialement l'un par rapport à l'autre, le premier élément (212) étant accouplé de manière pivotante à un sommet distal (410) et le second élément (202) étant accouplé de manière pivotante à un sommet proximal (414), lesdits sommets étant formés par les premières entretoises (408a).

15. Dispositif prothétique implantable selon la revendication 14, le premier élément étant un élément externe (212) et le second élément étant un élément interne (202) qui est partiellement logé à l'intérieur de l'élément externe (212).
